# EUROPEAN PATENT SPECIFICATION

(11) **EP 0 979 098 B1**
(45) Date of publication and mention of the grant of the patent: **12.03.2003**
(21) Application number: 98918696.0
(22) Date of filing: 24.04.1998
(51) Int. Cl.: A61K 38/31

(54) **METHOD OF TREATING HYPERPROLACTINEMIA AND PROLACTINOMAS**
VERFAHREN ZUR BEHANDLUNG VON HYPERPROLAKTINÄMIA UND PROLAKTINOMEN
PROCEDE DE TRAITEMENT DE L'HYPERPROLACTINEMIE ET DES PROLACTINOMES

(30) Priority: 01.05.1997 US 45241 P; 06.05.1997 US 852221
(43) Date of publication of application: 16.02.2000
(73) Proprietor: Cedars-Sinai Medical Center, Los Angeles, CA 90048 (US); Biomeasure Incorporated, Milford, MA 01757-3650 (US)
(72) Inventor: MELMED, Shlomo, Los Angeles, CA 90035 (US); SHIMON, Ilan, Beverly Hills, CA 90211 (US); CULLER, Michael, D., Hopkinton, MA 01748 (US)
(74) Representative: Ritthaler, Wolfgang, Dr.rer.nat.Dipl.-Chem.
(86) International application number: US9808288
(87) International publication number: WO98050063

(56) References cited:
- EP-A- 0 203 031
- WO-A-97/11962
- I. SHIMON ET AL.: "SOMATOSTATIN RECEPTOR SUBTYPE SPECIFICITY IN HUMAN FETAL PITUITARY CULTURES: DIFFERENTIAL ROLE OF SSTR2 AND SSTR5 FOR GROWTH HORMONE, THYROID-STIMULATING HORMONE, AND PROLACTIN REGULATION." THE JOURNAL OF CLINICAL INVESTIGATION, vol. 99, no. 4, 15 February 1997, pages 789-798, XP002073099 NEW YORK, N.Y., US cited in the application
- I. SHIMON ET AL.: "SOMATOSTATIN RECEPTOR (SSTR) SUBTYPE-SELECTIVE ANALOGUES DIFFERENTIALLY SUPRESS IN VITRO GROWTH HORMONE AND PROLACTIN IN HUMAN PITUITARY ADENOMAS: NOVEL POTENTIAL THERAPY FOR FUNCTIONAL PITUITARY TUMORS." THE JOURNAL OF CLINICAL INVESTIGATION, vol. 100, no. 9, 1 November 1997, pages 2386-2392, XP002073100 NEW YORK, N.Y., US

## Description

### BACKGROUND OF THE INVENTION

### Field of the Invention

This invention relates to the field of hyperprolactinemias, and prolactinomas. More specifically, this invention relates to the use of somatostatin type-5 recptor agonists for the treatment of a subject afflicted with either condition, and to the lowering of prolactin levels and/or the size of the tumor by administration of a selected family of pharmaceuticals.

### Description of the Background

Prolactin hypersecretion, or hyperprolactinemia, may have anyone of a number of functional causes, including various neurogenic causes such as thoracic sensory nerve stimulation, stress, and psychogenic causes, various hypothalamic causes such as diffuse processes, granulomatous diseases, neoplasms, stalk section, empty sella, non-lactotropic cell pituitary tumors, and prostradiation treatment to sella, various pituitary causes such as prolactinomas and pituitary lactotropic cell hyperplasia, and various endocrine causes such as pregnancy, estrogen administration, hypothyroidism, and adrenal insufficiency. Prolactin hypersecretion may also be caused by the administration of drugs which impair dopamine secretion, such as psychotropic drugs, antihypertensive drugs, antiemetic drugs, H₂-receptor blockers, and opiates, among others.

Prolactinomas are pituitary tumors, in fact, the most common pituitary tumors, which are almost five times more common in women than in men. Some prolactinomas respond to, and may be treated with, the dopamine agonist, Bromocriptine. One-third of the patients afflicted with prolactinomas, however, do not respond to, and are therefore not treatable with, this drug. The latter are usually referred for pituitary surgery and/or, rarely, irradiation.

Somatostatin, also known as somatotropin release inhibiting factor or SRIF, is believed to exert its biological effect through a family of G-protein associated receptors provided with seven transmembrane domains. Somatostatin inhibits the secretion of growth hormone. Five subtypes of the somatostatin receptor (SSTR) have already been cloned, and named SSTR-1, SSTR-2, SSTR-3, SSTR-4, and SSTR-5. The SSTR-1, SSTR-2, and SSTR-5 receptors have been shown to be expressed by the human pituitary gland, and the SSTR-1, SSTR-2, SSTR-3 and SSTR-5 receptors by human pituitary adenomas.

A large number of somatostatin agonists have been synthesized, and shown to preferentially bind to selected subtypes of the somatostatin receptor (SSTR) (See, below). Up until now, only SSTR-2 selective somatostatin agonists were shown to inhibit prolactin secretion from normal fetal pituitary cultures, as it is described e. g. in J. Clin. Invest. 99, 789-798 (1997).

Up to the present time, thus, it was not known which subtype(s) of the SSTR mediate(s) the regulation of prolactin production and secretion in the state of pathological hyperprolactinemia, such as that resulting, for example, from a prolactinoma.

### SUMMARY OF THE INVENTION

The present invention relates to the use of a therapeutically effective amount of one or more somatostatin type-5 receptor (SSTR-5) agonist(s) according to claim 1, such as a somatostatin type-5 selective agonist for the manufacture of a medicament for treating hyperpmlactinemia and/or a prolactinoma in a subject which may be a mammal, such as a human. This administration, for example, lowers abnormally high levels of prolactin in the subject's blood. In addition, when treating a prolactinoma, the medicament, for example, lowers either tumor prolactin secretion and/or decreases tumor size, in the subject.

The present medicament is effective in subjects afflicted by hyperprolactenemia, and particularly those who have abnormally high levels of prolactin, for example, as a result of one or more of the following: a pituitary tumor such as a prolactinoma, hypothalamic and pituitary stalk disease, hyperthyroidism, postparrum lactation, decreased dopamine or dopamine inhibitory action, which may be caused by the administration of drugs such as opiates and psychotropic drugs, among others, stimuli that overcome the normal dopamixiergic inhibition such as the administration of estrogen, decreased clearance of prolactin such as in renal failure or cirrhosis, seizures, and chest wall trauma, among others. Given that the present family of drugs lowers prolactin levels, it also consequently, treats the galactorrhea, amenorrhea, infertility, impotence, and/or hypogonadism, which many times accompany it.

While it is possible for the somatostatin agonist to be administered as the pure or substantially pure compound, it may also be presented as a pharmaceutical composition, formulation or preparation. The composition of the invention comprises one or more of a selected class of somatostatin agonists, and one or more pharmaceutically acceptable carriers for the agonists, and optionally other therapeutic agents. The somatostatin agonist may be formulated for administration by various routes, including by a parenteral route such as an intravenous, subcutaneous, or intramuscular route, or by implantation of a sustained release formulation, among others. The carrier must be "acceptable" in that it has to be compatible with, and stabilizing of, the active ingredient(s) of the formulation, and not deleterious to the subject to be treated.

Other features and advantages of the invention will be apparent from the following description of the preferred embodiments and from the claims.

### DETAILED DESCRIPTION OF THE INVENTION

The present invention arose from a desire by the inventors to improve on prior art treatments for hyperprolactinemias associated with various causes, including prolactinomas. This invention, thus, relates to the discovery that SSTR-5 somatostatin agonists, but not SSTR-2 somatostatin agonists, are capable of reducing serum prolactin levels, and inhibiting prolactin secretion in a subject which is in a state of pathological hyperprolactinemia, such as in the case of a subject carrying a prolactinoma. This invention, therefore, provides a medicament for treatment of hyperprolactinemias in general, and of prolactinomas in particular, in animals, including mammals such as humans. The invention counters by the administration of a selected group of pharmaceuticals the conditions afflicting the subject, and lowers prolactin blood levels and/or tumor size.

The present inventors are providing the use of a therapeutically effective amount of one or more somatostatin type-5 receptor (SSTR-5) agonist(s) according to claim 1, such as a somatostatin type-5 selective agonist for the manufacture of a medicament for treating hyperprolactinemia and/or a prolactinoma in a subject.

"Therapeutic amounts of the agonists of the invention are those which decrease the pituitary secretion of prolactin, or reduce serum prolactin levels to within about the normal range. Preferably, the amount of the SSTR-5 agonist to be administered is intended to reduce prolactin serum levels to about 0 to 50 ng/ml blood, as detected by an immunoradiometric assay (IRMA), or in some instances even above 50 ng/ml, and more preferably below 70 ng/ml blood. Still a more preferred therapeutic amount of the agonists of the invention are those that permit lowering the prolactin serum levels to about 2 to 30 ng/ml blood, and more preferably to about 5 to about 20 ng/ml blood. It is, however, to be understood, that although many subjects afflicted with hyperprolactinemia are highly responsive to the treatment disclosed by the invention, they respond to different degrees. Thus, where in patients with a large, invasive tumors and high serum prolactin levels, reductions of 20-60% and more are highly desirable, they are not always attained. In fact, the responses may be highly variable, and particularly favorable in the case of large, invasive tumors, particularly those normally observed in males. It is generally desirable to lower the prolactin serum levels to within the ranges described above, which are normal prolactin levels. A "therapeutic amount" of the agonist of the invention for the treatment of prolactinomas is meant as that dose which reduces the size of the tumor by at least about 30%, more preferably by about more than about 50%, and still more preferably by more than about 80% or more. In some instances, however, there is a complete or almost complete disappearance of the tumor, as detected by MRI. In general, the present treatment affords a reduction of about 4-50% of large tumors, 4-5 cm in diameter. In smaller tumors, of about 1 cm in diameter, this treatment may attain a significant reduction in size, detectable to about 2 mm. In some instances, smaller tumors may be less responsive than larger tumors to the present treatment. Thus, the amount of drugs to be administered in this invention, which is considered to be therapeutically effective clearly depends upon the condition being treated, the route of administration chosen, and the specific activity of the compound used. This is a matter which is ultimately best decided by the attending physician or veterinarian. In one embodiment, the somatostatin agonist is to be administered to the subject until the subject's prolactin level stabilizes to within normal levels, such as when the prolactinoma has been removed or ablated. In another embodiment, the somatostatin agonist may be administered, if necessary, for the lifetime of the patient.

### Somatostatin and its Agonists

Somatostatin or SRIF has a 14 amino acid isoform (somatostatin-14) as well as a 28 amino acid isoform (somatostatin-28). See Wilson, J. & Foster, D., Williams Textbook of Endocrinology, p. 510 (7th. Ed., 1985). Somatostatin inhibits the secretion of growth hormone, and was originally isolated from the hypothalamus. See, Brazeau, et al., Science 179:77 (1973). The native somatostatin has a very short lasting in vivo effect, because it is rapidly inactivated by endo- and exo-peptidases. Many novel agonists of somatostatin, such as peptides and non-peptidic compounds, have been prepared in order to enhance the duration of effect, and biological activity of this hormone, and selectivity for one or more particular subtype of somatostatin receptor. Such somatostatin analogues are called "somatostatin agonists" for the purposes of this patent.

As already indicated, various somatostatin receptors (SSTRs) have been isolated, among them SSTR-1, SSTR-2, SSTR-3, SSTR-4, and SSTR-5. The somatostatin agonist, therefore, may be a SSTR-1 agonist, SSTR-2 agonist, SSTR-3 agonist, SSTR-4 agonist, or a SSTR-5 agonist.

By 'somatostatin type-5 receptor agonist' or SSTR-5 agonist, is meant a compound which (1) has a high binding affinity, e. g., Ki less than 5 nM, and preferably less than 1 nM, for human SSTR-5, for example, as defined by the receptor binding assay described below, and (2) decreases prolactin levels in a patient, for example, as shown by the biological assay described below. Examples of somatostatin agonists are those covered by formulae or those specifically recited in the publications set forth below.
Van Binst, G. et al., Peptide Research 5: 8 (1992);
Horvath, A., et al., Abstract, "Conformations of Somatostatin Analogs Having Antitumor Activity", 22nd. European peptide Symposium, September 13-19, 1992, Interlaken, Switzerland;

| | |
|---|---|
| EP Appl. No. P5 164 EU; | PCT Appl. WO 91/09056 (1991); |
| EP Appl. No. 0 363 589 A2(1990); | US Patent No. 4,904,642; |
| US Patent No. 4,871,717; | US Patent No. 4,853,371; |
| US Patent No. 4,725,577; | US Patent No. 4,684,620; |
| US Patent No. 4,650,787; | US Patent No. 4,603,120; |
| US Patent No. 4,585,755; | EP Appl. No.0 203 031 A2 (1986); |
| US Patent No. 4,522,813; | US Patent No. 4,486,415; |
| US Patent No. 4,485,101; | US Patent No. 4,435,385; |
| US Patent No. 4,395,403; | US Patent No. 4,369,179; |
| US Patent No. 4,360,516; | US Patent No. 4,358,439; |
| US Patent No. 4,328,214; | US Patent No. 4,316,890; |
| US Patent No. 4,310,518; | US Patent No. 4,291,022; |
| US Patent No. 4,238,481; | US Patent No. 4,235,886; |
| US Patent No. 4,224,199; | US Patent No. 4,211,693; |
| US Patent No. 4,190,648; | US Patent No. 4,146,612; |
| US Patent No. 4.133,782; | US Patent No. 5,506,339; |
| US Patent No. 4,261,885; | US Patent No. 4,728,638; |
| US Patent No. 4,282,143; | US Patent No. 4,215,039; |
| US Patent No. 4,209,426; | US Patent No. 4,190,575; |
| EP Patent No. 0 389 180; | EP Appl. No. 0 505 680 (1982); |
| EP Appl. No. 0 083 305 (1982); | EP Appl. No. 0 030 920 (1980); |
| PCT Appl. No. WO 88/05052; | PCT Appl. No. WO 90/12811; |
| PCT Appl. No. WO 97/01579; | PCT Appl. No. WO 91/18016; |
| UK Appl.No.GB 2,095,261 (1981); French Appl.No.FR2,522,655(1983) | |

Examples of SSTR-5 agonists include, but are not limited to the following: H-D-Phe-Cys-Tyr-D-Trp-Lys-Val-Cys-β-Nal-NH2; H-D-Phe-Cys-Tyr-D-Trp-Lys-Thr-Cys-β-Nal-NH₂; and

H-D-Phe-Cys-Tyr(I)-D-Trp-Lys-Val-Cys-Thr-NH2.

By 'somatostatin type-5 receptor selective agonist is meant a somatostatin agonist which (1) has a higher binding affinity, or Ki, for SSTR-5 than for either SSTR-1, SSTR-2, SSTR-3, or SSTR-4, and (2) decreases prolactin levels in a patient, for instance, as shown by the biological assay described below. In one embodiment, the SSTR-5 selective agonist has a Ki for SSTR-5 that is at least 2 times, and may be at least 10 times, less than its Ki for the SSTR-2 receptor. For example, as defined by the receptor binding assay described below. In another embodiment, the somatostatin type-5 receptor selective agonist is also a SSTR-5 agonist. Examples of SSTR-5 selective somatostatin agonists include, but are not limited to the following somatostatin analogs, which are disclosed in the above-cited references: H-Cys-Phe-Phe-D-Trp-Lys-Thr-Phe-Cys-NH2 (BIM-23268); H-D-Phe-Phe-Phe-D-Trp-Lys-Thr-Phe-Thr-NH2 (BIM-23052); H-D-Phe-Phe-Tyr-D-Trp-Lys-Val-Phe-Thr-NH2; H-D-Tyr-Phe-Phe-D-Trp-Lys-Thr-Phe-Thr-NH2; H-D-Phe-Phe-Trp-D-Trp-Lys-Thr-Phe-Thr-NH2; H-D-Tyr-D-Phe-Tyr-D-Trp-Lys-Thr-Phe-Thr-NH2; H-D-Phe-Cpa-Tyr-D-Trp-Lys-Thr-Phe-Thr-NH2; H-D-Cys-Phe-Phe-D-Trp-Lys-Thr-Phe-Cys-NH2; H-Cys-Phe-Trp-D-Trp-Lys-Ser-Phe-Cys-NH2; H-Cys-Phe-Phe-D-Trp-Lys-Ser-Phe-Cys-NH2; H-Cys-Phe-Phe-D-Trp-Lys-Thr-Phe-Cys-NH2; H-Cys-Phe-Tyr-D-Trp-Lys-Thr-Phe-Cys-NH2; H-Cys-Phe-His-D-Trp-Lys-Thr-Phe-Cys-NH2; and

H-Cys-Phe-Tyr(I)-D-Trp-Lys-Thr-Phe-Cys-NH2.

It should be noted that for all somatostatin agonists represented above, each amino acid residue represents the structure of -NH-C(R)H-CO-, wherein R is the side chain of the amino acid (e.g., CH₃ for Ala). Lines between amino acid residues represent peptide bonds which join the amino acids. In addition, where the amino acid residue is optically active, it is the L-form configuration that is intended unless D-form is expressly designated. The abbreviations Cpa, β-Nal, and Tyr(I) stand for the amino acids p-Cl-Phe, 2-napthylalanine, and iodinated tyrosine (e.g., 3-I-Tyr, 5-I-Tyr, or 3, 5-I-Tyr). Although a disulfide bridge forms between two free thiols of the Cys residues, this is not shown above. Preferred are the peptides BIM-23268 and BIM-23052, as well as their mixture or combination. However, any of the other agonists listed above are also within the four corners of this invention.

The present agonists may be administered alone, or in a composition, or separately but jointly with other therapeutic agents, including other prolactin inhibitors. In addition to lowering levels of prolactin in the subject's blood, when administered to a subject afflicted with a prolactinoma, the present method is very effective in lowering either tumor prolactin secretion and/or in decreasing tumor size, or both. The present treatment is, thus, highly effective in subjects afflicted by abnormally high levels of prolactin caused by, for example, one or more of the following conditions: a pituitary tumor such as a prolactinoma, hypothalamic and pituitary stalk disease, hyperthyroidism, postpartum lactation, decreased dopamine or dopamine inhibitory action, which may be caused by the administration of drugs such as opiates and psychotropic drugs, among others, stimuli that overcome the normal dopaminergic inhibition such as the administration of estrogen, decreased clearance of prolactin such as in renal failure or cirrhosis, seizures, and chest wall trauma, among others.

In addition to lowering serum prolactin levels, the present family of drugs is also effective in the treatment of galactorrhea, amenorrhea, infertility, impotence, and/or hypogonadism, which many times are associated with increased prolactin levels.

The somatostatin agonist(s) of this invention may be administered as a purified, or substantially purified compound, it may also be presented as a pharmaceutical composition, formulation or preparation. The composition used according to the invention comprises one or more of a selected class of somatostatin agonists, and one or more pharmaceutically acceptable carriers for the agonists, and optionally other therapeutic agents. Formulations of the somatostatin agonist(s) described herein encompass those custom tailored for administration by various routes, including by a parenteral route, which may be an oral, intravenous, subcutaneous, or intramuscular route, or by implantation of a sustained release formulation, among others. The carrier(s) must be "acceptable" in that it has to be compatible with, and stabilizing of, the active ingredient(s) of the formulation, and not deleterious to the subject to be treated ("pharmaceutically acceptable").

The formulation should preferably be free of oxidizing agents, or other substances with which peptides are known to be incompatible. Somatostatin agonists that are in their cyclized form, such as those that have an internal cysteine disulfide bond may, for example, be in their oxidized or reduced forms. Thus, the presence of a reducing agent in the composition might lead to an opening of the cysteine disulfide bridge. On the other hand, highly oxidative conditions may, for example, lead to the formation of cysteine sulfoxide, and to the oxidation of tryptophan. It is, therefore, of great importance that excipients and other components of the formulation be carefully select. The pH of the composition is also of import, and it is preferable that the product be buffered under slightly acidic conditions, such as pH about 5 to about 6. These pHs, however, are not limiting.

In general, the formulations for tablets or powders may be prepared by uniformly and intimately blending the active ingredient with finely divided solid carriers, and then, if necessary, as in the case of tablets, forming the product into the desired shape and size.

Formulations suitable for parenteral, e.g., intravenous, administration, on the other hand, conveniently comprise sterile aqueous solutions of the active ingredient(s). The solutions are, preferably, isotonic with the blood of the subject to be treated. Such formulations may. For example, be conveniently prepared by dissolving solid active ingredient(s) in water or another aqueous solvent to produce an aqueous solution, and rendering the solution sterile. These and other formulation may be presented in unit or multidose form, in containers, for example, sealed ampoules or vials, among others. The formulation may be prepared by any of the well known methods to the pharmaceutical artisan. All methods include the step of bringing the active ingredient(s) into association with the carrier, which constitutes one or more accessory ingredients. Sustained release formulations suitable for parenteral administrations, such as biodegradable polymer formulations, for example, those including polyesters containing lactic or glycolic acid residues, are also well known in the art. See, e.g., U.S. Patent Nos. 3,773,919 and 4,767,628 and PCT Publication No. WO 94/15587.

The somatostatin agonist used according to the invention or its composition may also be administered with another compound capable of lowering blood levels of prolactin, including dopamine agents such as Bromocriptine, cabergoline, pergolide, or lisuride, among others. This compounds may be added to the same composition, or administered separately in amounts that are known in the art. In some instances, when administered in joint therapy with the agonist(s) of this invention, the amounts of the latter compounds administered will be lower than the doses which are utilized when they are administered by themselves. In other instances, however, similar doses may be utilized, which will be additive or potentiating of the present treatment.

The somatostatin agonist may be injected parenterally, e.g., intravenously, into the bloodstream of the subject being treated. It will, however, be readily appreciated by those skilled in the art that which route, whether intravenous, subcutaneous, intramuscular, intraperitoneal, enterally, transdermally, transmucously, sustained released polymer compositions, e. g., lactic acid polymer or lactic acid and glycolic acid copolymer microparticle or implant, perfusion, nasal, oral, etc., will depend on the condition being treated, and the activity and bioavailability of the somatostatin agonist being used.

It is believed that one skilled in the art may, based on the description provided herein, utilize the present invention to its fullest extent. The following specific embodiments are, therefore, to be construed as merely illustrative, and not limiting of the remainder of the disclosure in any way or manner.

Unless defined otherwise, all technical and scientific terms used herein have the same meaning as commonly understood by one of ordinary skill in the art to which this invention belongs.

The invention will now be described with reference to some specific examples, which are representative of the invention in general. These examples are solely exemplary, and not limiting of the invention in any form or mode.

### EXAMPLES

### Example 1: Synthesis of BIM-23052

The peptide H-D-Phe-Phe-Phe-D-Trp-Lys-Thr-Phe-Thr-NH2 (BIM-23052) described above may be synthesized by following the protocol set forth in Example I of European Pat. Appl. 0 395 417 A1.

### Example 2: Synthesis of other Somatostatin Agonists

The methods for synthesizing somatostatin agonists are well documented and are within the ability of a person of ordinary skill in the art. In addition, the synthesis of short amino acid sequences is well established in the peptide art. The syntheses of cyclic somatostatin analogs are described earlier cited patents/patent applications referenced in the prior subsection (e.g., U.S. Patent No. 4,904,642). The synthesis of somatostatin agonists with a substituted N-terminus may be achieved, for example, by following the protocol set forth in WO 88/02756, European Pat. Appl. No. 0 329 295, and PCT Publication No. WO 94/04752.

### Example 3: Isolation of Somatostatin Receptor DNA

The human SSTR-1, SSTR-2, SSTR-3, SSTR-4, and SSTR-5 cDNA clones have been described (SSTR-1 and SSTR-2: Yamada, Y., et al., P.N.A.S. USA, 89: 251-255 (1992); SSTR-3: Yamada, et al., Mol. Endocrinol. 6: 2136-2142 (1993); and SSTR-4 and SSTR-5: Yamada, et al., B.B.R.C. 195: 844-852 (1993)). The actual clones for the SSTR-1 to -3 are available from the American Type Culture Collection (ATCC, Rockville, MD), under the Accesion Nos. ATCC Nos. 79044 (SSTR-1), 79046 (SSTR-2), and 79048 (SSTR-3)).

Based on the restriction endonuclease maps, the entire coding region of each SSTR cDNA may be excised by suitable restriction endonuclease digestion (See, Maniatis, T., et al., Molecular Cloning-A Laboratory Manual, CSHL, (1982)). Restriction endonucleases are available, e.g., from New England Biolabs (Beverly, MA). The present cDNA fragment was inserted into the mammalian expression vector, pCMV (Russell, D., et al., J. Biol. Chem., 264:8222-8229 (1989)), using standard molecular biology techniques (See, e.g., Maniatis, T., et al., Molecular Cloning,-A Laboratory Manual, Cold Spring Harbor Laboratory (1982)), to produce the expression plasmid, pCMV-human SSTR-1 through pCMV-human SSTR-5.

Other mammalian expression vectors suitable for use herein include pcDNA1/Amp (Invitrogen, Sandlesy, CA). The expression plasmids were introduced into the suitable bacterial host, E. Coli HB101 (Stratagene, La Jolla, CA) and plasmid DNAs, for transfection, were prepared on Cesium Chloride gradients.

### Example 4: Cell Transfection with Hybrid Vector

CHO-K1 (ovary, Chinese hamster) cells were obtained from ATCC (ATCC No. CCL 61). The cells were grown and maintained in Ham's F12 media (Gibco BRL, Grand Island, NY) supplemented with 10% fetal bovine serum under standard tissue culture conditions. For transfection, the cells were seeded at a density 1 x 10⁶/60-cm plate (Baxter Scientific Products, McGaw Park, IL.). DNA mediated transfection was carried out using the calcium phosphate co-precipitation method (Ausubel, F.M., et al., Current Protocols in Molecular Biology, John Wiley & Sons, 1987). The plasmid pRSV-neo (ATCC; ATCC No. 37198) was included as a selectable marker at 1/10 the concentration of the expression plasmid. CHO-K1 clonal cell lines that have stably inherited the transfected DNA were selected for growth in Ham's F12 media containing 10% fetal bovine serum and 0.5 mg/ml of G418 (Sigma). The cells were ring-cloned and expanded in the same media for analysis.

### Example 5: Cell Expression of Somatostatin Receptors

Expression of the human SSTR-1 through SSTR-5 receptors in the CHO-K1 cells were detected by Northern blot analysis of total RNA prepared from the cells (Sambrook, J. E., et al., Molecular Cloning - A Laboratory Manual, Ed. 2., Cold Spring Harbor Laboratory, Cold Spring Harbor, NY, 1989) and by receptor binding using [¹²⁵I-Tyr¹¹] somatostatin-14 as a ligand. Transfected cell lines expressing the human SSTR receptors were clonally expanded in culture and used in the following SSTR binding protocol.

### Example 6: Preparation of Receptor-containing Membranes

Crude membranes were prepared by homogenization of the transfected cells in 20 ml of ice-cold 50 mM Tris-HCl with a POLYTRON homogenizer (setting 6, 15 sec). Buffer was added to obtain a final volume of 40 ml, and the homogenate was centrifuged in a Sorval SS-34 rotor at 39,000 g for 10 min at 0-4°C. The resulting supernatant was decanted and discarded. The pellet was re-homogenized in ice-cold buffer, diluted, and centrifuged as before. The final pellet was resuspended in the 10 mM Tris HCl and held on ice for the receptor binding assay.

### Example 7: Somatostatin Receptor Binding Assay

Aliquots of the membrane preparation were incubated for 30 min at 30 °C with 0.05 nM [¹²⁵I-Tyr11] somatostatin-14 (2000 Ci/mmol; Amersham Corp., Arlington Heights, IL) in 50 mM HEPES (pH 7.4) containing a test somatostatin agonist of various concentrations (e.g., 10⁻¹¹ to 10⁻⁶), 10 mg/ml bovine serum albumin (fraction V) (Sigma Chemical Co., St. Louis, MO), MgCl₂ (5 mM), Trasylol (200 KIU ml), bacitracin (0.02 mg/ml), and phenylmethylsulphonyl fluoride (0.02 mg/ml). The final assay volume was 0.3 ml. The incubations were terminated by rapid filtration through GF/C filters (pre-soaked in 0.3% polyethylenimine for 30 min) using a Brandel filtration manifold. Each tube and filter were then washed three times with 5 ml aliquots of ice-cold buffer.

Specific binding is defined as the total [¹²⁵I-Tyr¹¹]SRIF-14 bound minus that bound in the presence of 1000 nM. The Ki values for the tested somatostatin agonists were calculated by using the following formula: Ki = IC₅₀ /[1 +(LC/LEC)] where IC₅₀ is the concentration of test somatostatin agonist required to inhibit 50 percent of the specific binding of the radioligand [¹²⁵I-Tyr11] somatostatin-14, LC is the concentration of the radioligand (0.05 nM), and LEC is the equilibrium dissociation constant of the radioligand (0.16 nM). The Ki values (nm) for the tested somatostatin agonists are shown in Table I below.

**Table I:**

| **Ki (nm) for Selected Somatostatin Agonists** | | | | | |
|---|---|---|---|---|---|
| | hSSTR-1 | hSSTR-2 | hSSTR-3 | hSSTR-4 | hSSTR-5 |
| Somatostatin-14 | 2.26 | 0.23 | 1.2 | 1.8 | 1.41 |
| Somatostatin-28 | 2.38 | 0.30 | 1.3 | 7.93 | 0.4 |
| Lanreotide | 2414 | 0.75 | 97.9 | 1826 | 5.21 |
| BIM-23268 | 1227 | 15.06 | 545 | 3551 | 0.42 |
| BIM-23052 | 97.6 | 11.96 | 5.6 | 127 | 1.22 |
| Octreotide | 875 | 0.57 | 26.8 | 5029 | 6.78 |
| BIM-23190 | 9120 | 0.35 | 215 | 7537 | 11.1 |
| BIM-23197 | 6016 | 0.19 | 26.8 | 3897 | 9.81 |
| BIM-23023 | 6616 | 0.43 | 869 | 2700 | 4.18 |
| * Subtype affinity was determined by radioligand membrane receptor binding assays in CHO cells expressing human SSTR2 gene or SSTR -5 cDNA, as described by Shimon, I., et al., J. Clin. Invest. 99:789 (1997). | | | | | |

### Example 8: Reduction of Prolactin Secretion from Prolactinomas

Cells derived from six prolactin-secreting pituitary adenomas were treated with somatostatin agonists with different binding selectivities for SSTR-2 and SSTR-5. Tumor specimens were cultured as described as described by Shimon, I. et al. (1997) (See, Table II below). Briefly, the specimens were washed in DME with 0.3% BSA, minced and enzymatically dissociated using 0.35% collagenase and 0.1% hyaluronidase (Sigma Chemical Co., St. Louis, MO) for 1 hour. The cell suspensions were filtered and resuspended in low glucose DME with 10% FBS and antibiotics before seeding in 48-well tissue culture plates (∼5 x 104 cells/well; 50-80 wells/tumor) in 0.5 ml medium for 96 hours.

The medium was changed before treatment to serum-free defined DME, and the cell cultures were treated for 3 hours with 10-100 nM of the type 2-selective somatostatin agonists BIM-23014 (Lanreotide), BIM-23023, BIM-23190, and BIM-23197, the sequences of which are disclosed in Moreau, et al., Metabolism 45(8):24 (1996), and the type-5 selective somatostatin agonists BIM-23268 and BIM-23052. (6-8 wells for each analog or control (vehicle solution only)), after which medium was frozen for later hormone measurements.

Human prolactin (PRL) levels were measured by immunoradiometric assay (obtained from Diagnostic Products Corporation, Los Angeles, CA) after appropriate sample dilution, as described by Shimon I., et al., J. Clin. Invest. 99:789-798 (1997).

### Example 9: Treatment of Adenoma Cells with Somatostatin Type-5 Agonists in Accordance with the Invention

Six prolactin-secreting pituitary adenomas were obtained, cultured, and treated with analogues representative of the somatostatin analogues of the invention. Adenoma cells were obtained from large macroprolactinomas of 4 male patients (Patient Tumors 1, 2, 3, and 4). Three of the macroprolactinomas were invasive, and were accompanied by serum prolactin levels over 1,000 ng/ml. The cells from all 4 different tumors responded to treatment with the SSTR-5 selective agonists BIM-23052 or BIM-23268 with suppression of prolactin secretion.

The SSTR-5 type selective somatostatin agonists BIM-23052 and BIM-23268 (10 nM) suppressed prolactin secretion by 33 and 36%, respectively (p<0.05), in cell cultures from tumor 1. In contrast, the SSTR-2 selective somatostatin agonists Laureotide, BIM-23023, BIM-23190, and BIM-23197, did not affect prolactin release from the adenomatous cell cultures of tumor 1.

Prolactin release from tumor 2 cell cultures were inhibited by treatment with 10 nM BIM-23268 (38% versus controls; p < 0.0005).

Large tumor 3 cell cultures were treated with 10 nM and 100 nM of the SSTR-5 selective somatostatin agonist BIM-23268. The type-5 agonist BIM-23268 suppressed prolactin in a dose dependent manner by up to 29% (p < 0.05).

Large tumor 4 cell cultures suppressed prolactin release in response to both BIM-23052, and BIM-23268 by more than 30% (p≺0.05). whereas BIM-23190, and BIM-23197 modestly stimulated prolactin production.

The four large male tumors also failed to respond to commercially available drugs, such as Octrotide, and Lanreotide, as well as to novel agonists with even higher affinities for the SSTR-2, including BIM-23190, and BIM-23197.

Small non-invasive prolactin-secreting tumors were obtained from female patients 5 and 6, who had circulating prolactin levels of less than 200 ng/ml serum. These cells failed to significantly respond to any of the somatostatin agonists examined. The clinical characteristics of the patients are shown in Table III below.

**Table III:**

| **Clinical Characteristics of Six Prolactinoma Patients** | | | | | | | |
|---|---|---|---|---|---|---|---|
| **No.** | **Sex** | **Age** | **PRL** | **Tumor Size** | **DA** | **Indication for** | **In vitro** **SSTR5** |
| | | | **ng/ml** | **(cm)** | **Therapy** | **surgery** | **Response** |
| **1** | M | 64 | 742 | 1.5 non-invasive | none | patient preference | + |
| **2** | M | 33 | 1249 | 3.0 invasive | bromocriptirie | drug compliance | + |
| **3** | M | 41 | 1514 | > 3.0 invasive | bromocriptine | DA resistance | + |
| **4** | M | 33 | 2862 | 3.4 invasive | bromocriptine | DA resistance | + |
| **5** | F | 29 | 67 | 2.7 non-invasive | none | patient preference | - |
| **6** | F | 27 | 150 | 0.9 non-invasive | bromocriptine | DA resistance | - |
| DA, dopamine agonist. | | | | | | | |

Having now described the invention in general, and by reference to specific examples, it is understood that many variations of the above may be practiced by an artisan based on the above disclosure, which are within the scope of the invention, and of the following claims. The foregoing description has been limited to specific embodiments of this invention. It will be apparent, however, that these variations and modifications made to the invention, with the ensuing attainment of some or all of the advantages of the inventions, are also within the scope of the following claims.

## Claims

1. Use of a therapeutically effective amount of a somatostatin type-5 receptor selective agonist for the manufacture of a medicament for treating hyperprolactinemia in a subject,
**characterized in that**
the type-5 receptor agonist comprises a peptide selected from the group consisting of H-Cys-Phe-Phe-D-Trp-Lys-Thr-Phe-Cys-NH₂, H-D-Phe-Phe-Phe-D-Trp-Lys-Thr-Phe-Thr-NH₂, and mixtures thereof; and
the amount of the agonist to be administered is effective to lower prolactin levels to within 0 to 50 ng/ml blood, when measured by immunoradiometry.

2. The use of claim 1, wherein the somatostatin type-5 receptor agonist has a Ki less than 1 nM for the somatostatin type-5 receptor.

3. The use of claim 1, wherein the Ki of the somatostatin type-5 receptor selective agonist for the type-5 somatostatin receptor is at least 10 times lower than its Ki for the somatostatin type-2 receptor.

4. The use of claims 1 to 3, wherein the type-5 receptor selective agonist comprises H-Cys-Phe-Phe-D-Trp-Lys-Thr-Phe-Cys-NH₂.

5. The use of claim 1 to 3, wherein the type-5 receptor selective agonist comprises H-D-Phe-Phe-Phe-D-Trp-Lys-Thr-Phe-Thr-NH₂.

6. The use of claim 1, wherein the amount of the agonist to be administered is effective to lower prolactin levels to within about 2 to about 30 ng/ml blood, when measured by immunoradiometry.

7. The use of claim 1, wherein the amount of the agonist to be administered is effective to lower prolactin levels to within about 5 to about 20 ng/ml blood, when measured by immunoradiometry.

8. The use of claim 1, wherein the patient is one being administered an amount of a therapeutic agent selected from the group consisting of Bromocriptine, cabergoline, pergolide, lisoride, and combinations thereof.

9. Use of a therapeutically effective amount of a somatostatin type-5 receptor agonist for the manufacture of a medicament for treating a subject afflicted by hyperprolactinemia associated with postpartum lactation.

10. Use of a therapeutically effective amount of a somatostatin type-5 receptor agonist for the manufacture of a medicament for treating a subject afflicted by hyperprolactinemia induced by drugs.

11. The use of claim 10, wherein the drug is selected from the group consisting of opiates and psychotropic drugs.

12. Use of a therapeutically effective amount of a somatostatin type-5 receptor agonist for the manufacture of a medicament for treating a subject afflicted by hypothalamic or pituitary stalk disease associated with high blood levels of prolactin.

13. Use of a therapeutically effective amount of a somatostatin type-5 receptor agonist for the manufacture of a medicament for treating a subject afflicted by hyperthyroidism associated with high levels of serum prolactin.

14. Use of a therapeutically effective amount of a somatostatin type-5 receptor agonist for the manufacture of a medicament for treating a subject afflicted by decreased dopamine or dopamine inhibitory action associated with high levels of serum prolactin.

15. Use of a therapeutically effective amount of a somatostatin type-5 receptor agonist for the manufacture of a medicament for treating a subject afflicted by a stimulus that overcomes normal dopaminergic inhibition associated with high levels of serum prolactin.

16. The use of claim 15, wherein the stimulus that overcomes normal dopaminergic inhibition comprises the administration of estrogen.

17. Use of a therapeutically effective amount of a somatostatin type-5 receptor agonist for the manufacture of a medicament for treating a subject afflicted by decreased clearance of prolactin associated with high levels of serum prolactin.

18. Use of a therapeutically effective amount of a somatostatin type-5 receptor agonist for the manufacture of a medicament for treating a subject afflicted by renal failure or cirrhosis associated with high levels of serum prolactin.

19. Use of a therapeutically effective amount of a somatostatin type-5 receptor agonist for the manufacture of a medicament for treating a subject afflicted by seizures associated with high levels of serum prolactin.

20. Use of a therapeutically effective amount of a somatostatin type-5 receptor agonist for the manufacture of a medicament for treating a subject afflicted by chest wall trauma associated with high levels of serum prolactin.

21. The use of claim 1, wherein the subject is a human.

22. The use of claim 1, wherein the subject is afflicted by a prolactinoma.

23. The use of claim 22, wherein the amount of the somatostatin type-5 receptor agonist is effective for lowering the amount of prolactin secreted by the prolactinoma to maintain within about normal blood levels of prolactin.

24. The use of claim 22, wherein the amount of the agonist to be administered is effective to lower prolactin levels to within about 2 to about 30 ng/ml blood, when measured by immunoradiometry.

25. The use of claim 22, wherein the amount of the somatostatin type-5 receptor agonist is effective for lowering the prolactin levels to within about 5 to about 20 ng/ml blood, when measured by immunoradiometry.

26. The use of claim 22, wherein the somatostatin type-5 receptor agonist has a Ki for the somatostatin type-5 receptor that is less than 1 nM.

27. The use of claim 22, wherein the Ki of the somatostatin type-5 receptor selective agonist for the type-5 somatostatin receptor is at least 10 times lower than its Ki for the somatostatin type-2 receptor.

28. Use of a therapeutically effective amount of a somatostatin type-5 receptor selective agonist for the manufacture of a medicament for treating galactorrhea associated with high levels of prolactin.

29. Use of a therapeutically effective amount of a somatostatin type-5 receptor selective agonist for the manufacture of a medicament for treating amenorrhea associated with high levels of prolactin.

30. Use of a therapeutically effective amount of a somatostatin type-5 receptor selective agonist for the manufacture of a medicament for treating infertility associated with high levels of prolactin.

31. Use of a therapeutically effective amount of a somatostatin type-5 receptor selective agonist for the manufacture of a medicament for treating impotence associated with high levels of prolactin.

32. Use of a therapeutically effective amount of a somatostatin type-5 receptor selective agonist for the manufacture of a medicament for treating hypergonadism associated with high levels of prolactin.

## Patentansprüche

1. Verwendung einer therapeutisch effektiven Menge eines Somatostatin Typ-5 Rezeptor-selektivem Agonisten für die Herstellung eines Medikamentes zum Behandeln von Hyperprolaktinämie in einem Subjekt,
**dadurch gekennzeichnet, daß**
der Typ-5 Rezeptor Agonist ein Peptid umfaßt, das ausgewählt ist aus der Gruppe bestehend aus H-Cys-Phe-Phe-D-Trp-Lys-Thr-Phe-Cys-NH₂, H-D-Phe-Phe-Phe-D-Trp-Lys-Thr-Phe-Thr-NH₂, und Mischungen daraus; und
die Menge des zu verabreichenden Agonisten effektiv darin ist, die Prolaktinspiegel bis auf 5 bis 50 ng/ml Blut abzusenken, wenn mit Immunoradiometrie gemessen wird.

2. Die Verwendung nach Anspruch 1, wobei der Somatostatin Typ-5 Rezeptor Agonist einen Ki kleiner als 1 nM für den Somatostatin Typ-5 Rezeptor hat.

3. Die Verwendung nach Anspruch 1, wobei der Ki des Somatostatin Typ-5 Rezeptor-selektiven Agonisten für den Typ-5 Somatostatin Rezeptor wenigstens zehnmal niedriger ist, als sein Ki für den Somatostatin Typ-2 Rezeptor.

4. Die Verwendung nach Anspruch 1 bis 3, wobei der Typ-5 Rezeptor-selektive Agonist H-Cys-Phe-Phe-D-Trp-Lys-Thr-Phe-Cys-NH₂ umfaßt.

5. Die Verwendung nach Anspruch 1 bis 3, wobei der Typ-5 Rezeptor-selektive Agonist H-D-Phe-Phe-Phe-D-Trp-Lys-Thr-Phe-Thr-NH₂ umfaßt.

6. Die Verwendung nach Anspruch 1, wobei die Menge des Agonisten, der verabreicht wird, darin effektiv ist, die Prolaktinspiegel auf etwa 2 bis zu etwa 30 ng/ml Blut zu senken, wenn mit Immunoradiometrie gemessen wird.

7. Die Verwendung nach Anspruch 1, wobei die Menge des zu verabreichenden Agonisten darin effektiv ist, die Prolaktinspiegel auf etwa 5 bis zu etwa 20 ng/ml Blut zu senken, wenn mit Immunoradiometrie gemessen wird.

8. Die Verwendung nach Anspruch 1, wobei der Patient einer ist, dem eine Menge eines therapeutischen Mittels ausgewählt aus der Gruppe bestehend aus Bromocriptin, Cabergolin, Pergolid, Lisorid und Kombinationen daraus verabreicht wird.

9. Die Verwendung einer therapeutisch effektiven Menge eines Somatostatin Typ-5 Rezeptor Agonisten für die Herstellung eines Medikamentes zur Behandlung eines Subjektes, das unter Hyperprolaktinämie verbunden mit Laktation nach der Geburt leidet.

10. Die Verwendung einer therapeutisch effektiven Menge eines Somatostatin Typ-5 Rezeptor Agonisten für die Herstellung eines Medikamentes zur Behandlung eines Subjektes, das unter durch Drogen hervorgerufener Hyperprolaktinämie leidet.

11. Die Verwendung nach Anspruch 10, wobei die Droge ausgewählt ist aus der Gruppe bestehend aus Opiaten und psychotropischen Drogen.

12. Die Verwendung einer therapeutisch effektiven Menge eines Somatostatin Typ-5 Rezeptor Agonisten für die Herstellung eines Medikamentes zur Behandlung eines Subjektes, das an hypothalamischen oder hypophysären Stepiteserkrankungen (stalk disease), verbunden mit hohen Blutspiegeln an Prolaktin, leidet.

13. Die Verwendung einer therapeutisch effektiven Menge eines Somatostatin Typ-5 Rezeptor Agonisten für die Herstellung eines Medikamentes zur Behandlung eines Subjektes, das an Hyperthyroidismus, verbunden mit hohen Spiegeln an Serumprolaktin, leidet.

14. Die Verwendung einer therapeutisch effektiven Menge eines Somatostatin Typ-5 Rezeptor Agonisten für die Herstellung eines Medikamentes zur Behandlung eines Subjektes, das an verminderter Dopamin- oder Dopamin-Inhibitoraktion verbunden mit hohen Spiegeln an Serumprolaktin leidet.

15. Die Verwendung einer therapeutisch effektiven Menge eines Somatostatin Typ-5 Rezeptor Agonisten für die Herstellung eines Medikamentes zur Behandlung eines Subjektes, das an einem Stimulus leidet, der normale dopaminergische Inhibition überwindet, in Verbindung mit hohen Spiegeln an Serumprolaktin.

16. Die Verwendung nach Anspruch 15, wobei der Stimulus, der die normale dopaminergische Inhibition überwindet, die Verabreichung von Östrogen umfaßt.

17. Die Verwendung einer therapeutisch effektiven Menge eines Somatostatin Typ-5 Rezeptor Agonisten für die Herstellung eines Medikamentes zur Behandlung eines Subjektes, das an verminderter Prolaktinentfernung leidet, verbunden mit hohen Spiegeln an Serumoprolaktin.

18. Die Verwendung einer therapeutisch effektiven Menge eines Somatostatin Typ-5 Rezeptor Agonisten für die Herstellung eines Medikamentes zur Behandlung eines Subjektes, das an Nierenversagen oder Zirrhose leidet, verbunden mit hohen Spiegeln an Serumprolaktin.

19. Die Verwendung einer therapeutisch effektiven Menge eines Somatostatin Typ-5 Rezeptor Agonisten für die Herstellung eines Medikamentes zur Behandlung eines Subjektes, das an Anfällen leidet, die mit hohen Serumprolaktinspiegeln verbunden sind.

20. Die Verwendung einer therapeutisch effektiven Menge eines Somatostatin Typ-5 Rezeptor Agonisten für die Herstellung eines Medikamentes zur Behandlung eines Subjektes, das an einem Brustwandtrauma leidet, verbunden mit hohen Spiegeln an Serumprolaktin.

21. Die Verwendung nach Anspruch 1, wobei das Subjekt menschlich ist.

22. Die Verwendung nach Anspruch 1, wobei das Subjekt an Prolaktinoma leidet.

23. Die Verwendung nach Anspruch 22, wobei die Menge des Somatostatin Typ-5 Rezeptor Agonisten darin effektiv ist, die Menge an Prolaktin, das durch die Prolaktinoma abgesondert wird, abzusenken, um es in etwa innerhalb der normalen Prolaktin-Blutspiegel zu halten.

24. Die Verwendung nach Anspruch 22, wobei die Menge des zu verabreichenden Agonisten darin effektiv ist, die Prolaktinspiegel bis zu etwa 2 bis 30 ng/ml Blut abzusenken, wenn durch Immunoradiometrie gemessen wird.

25. Die Verwendung nach Anspruch 22, wobei die Menge des Somatostatin Typ-5 Rezeptor Agonisten darin effektiv ist, die Prolaktinspiegel bis zu etwa 5 bis 20 ng/ml Blut abzusenken, wenn durch Immunoradiometrie gemessen wird.

26. Die Verwendung nach Anspruch 22, wobei der Somatostatin Typ-5 Rezeptor Agonist einen Ki für den Somatostatin Typ-5 Rezeptor hat, der kleiner ist als 1 nM.

27. Die Verwendung nach Anspruch 22, wobei der Ki des Somatostatin Typ-5 Rezeptor-selektivem Agonisten für den Typ-5 Somatostatin Rezeptor wenigstens zehnmal niedriger ist, als sein Ki für den Somatostatin Typ-2 Rezeptor.

28. Die Verwendung einer therapeutisch effektiven Menge eines Somatostatin Typ-5 Rezeptor-selektivem Agonisten für die Herstellung eines Medikamentes zur Behandlung von Galaktorrhö verbunden mit hohen Prolaktinspiegeln.

29. Die Verwendung einer therapeutisch effektiven Menge eines Somatostatin Typ-5 Rezeptor-selektivem Agonisten für die Herstellung eines Medikamentes zur Behandlung von Amenorrhö, assoziiert mit hohen Prolaktinspiegeln.

30. Die Verwendung einer therapeutisch effektiven Menge eines Somatostatin Typ-5 Rezeptor-selektivem Agonisten für die Herstellung eines Medikamentes zur Behandlung von Unfruchtbarkeit, verbunden mit hohen Prolaktinspiegeln.

31. Die Verwendung einer therapeutisch effektiven Menge eines Somatostatin Typ-5 Rezeptor-selektivem Agonisten für die Herstellung eines Medikamentes zur Behandlung von Impotenz, verbunden mit hohen Prolaktinspiegeln.

32. Die Verwendung einer therapeutisch effektiven Menge eines Somatostatin Typ-5 Rezeptor-selektivem Agonisten für die Herstellung eines Medikamentes zur Behandlung von Hypergonadismus, verbunden mit hohen Prolaktinspiegeln.

## Revendications

1. Utilisation d'une quantité efficace d'un point de vue thérapeutique d'un agoniste sélectif du récepteur de somatostatine de type 5 pour la fabrication d'un médicament pour le traitement de l'hyperprolactinémie chez un sujet,
**caractérisée en ce que**
l'agoniste du récepteur de type 5 comprend un peptide sélectionné parmi le groupe constitué de H-Cys-Phe-Phe-D-Trp-Lys-Thr-Phe-Cys-NH₂, H-D-Phe-Phe-Phe-D-Trp-Lys-Thr-Phe-Thr-NH₂ et des mélanges de ceux-ci ; et
la quantité d'agoniste à administrer est efficace pour réduire les taux de prolactine dans la fourchette de 0 à 50 ng/ml de sang, lorsque mesurés par radiométrie immunologique.

2. Utilisation selon la revendication 1, dans laquelle l'agoniste du récepteur de somatostatine de type 5 présente un Ki de moins de 1 nM pour le récepteur de somatostatine de type 5.

3. Utilisation selon la revendication 1, dans laquelle le Ki de l'agoniste sélectif du récepteur de somatostatine de type 5 pour le récepteur de somatostatine de type 5 est au moins 10 fois inférieur au Ki pour le récepteur de somatostatine de type 2.

4. Utilisation selon les revendications 1 à 3, dans laquelle l'agoniste sélectif du récepteur de type 5 comprend le peptide H-Cys-Phe-Phe-D-Trp-Lys-Thr-Phe-Cys-NH₂.

5. Utilisation selon les revendications 1 à 3, dans laquelle l'agoniste sélectif du récepteur de somatostatine de type 5 comprend le peptide H-D-Phe-Phe-Phe-D-Trp-Lys-Thr-Phe-Thr-NH₂.

6. Utilisation selon la revendication 1, dans laquelle la quantité d'agoniste à administrer est efficace pour réduire les taux de prolactine dans la fourchette d'environ 2 à environ 30 ng/ml de sang, lorsque mesurés par radiométrie immunologique.

7. Utilisation selon la revendication 1, dans laquelle la quantité d'agoniste à administrer est efficace pour réduire les taux de prolactine dans la fourchette d'environ 5 à environ 20 ng/ml de sang, lorsque mesurés par radiométrie immunologique.

8. Utilisation selon la revendication 1, dans laquelle le patient est un patient auquel on administre une quantité d'un agent thérapeutique sélectionné parmi le groupe constitué de la bromocriptine, la cabergoline, la pergolide, la lisoride et des combinaisons de celles-ci.

9. Utilisation d'une quantité efficace d'un point de vue thérapeutique d'un agoniste du récepteur de somatostatine de type 5 pour la fabrication d'un médicament pour traiter un sujet atteint d'hyperprolactinémie associée à une lactation postpartum.

10. Utilisation d'une quantité efficace d'un point de vue thérapeutique d'un agoniste du récepteur de somatostatine de type 5 pour la fabrication d'un médicament pour traiter un sujet atteint d'hyperprolactinémie induite par des médicaments.

11. Utilisation selon la revendication 10, dans laquelle le médicament est sélectionné parmi le groupe constitué des opiacés et des médicaments psychotropes.

12. Utilisation d'une quantité efficace d'un point de vue thérapeutique d'un agoniste du récepteur de somatostatine de type 5 pour la fabrication d'un médicament pour traiter un sujet atteint de maladies hypothalamiques ou hypophysaires associées à des taux élevés de prolactine dans le sang.

13. Utilisation d'une quantité efficace d'un point de vue thérapeutique d'un agoniste du récepteur de somatostatine de type 5 pour la fabrication d'un médicament pour traiter un sujet atteint d'hyperthyroïdie associée à des taux élevés de prolactine dans le sérum.

14. Utilisation d'une quantité efficace d'un point de vue thérapeutique d'un agoniste du récepteur de somatostatine de type 5 pour la fabrication d'un médicament pour traiter un sujet atteint d'une baisse de la dopamine ou d'une action d'inhibition de la dopamine associée à des taux élevés de prolactine dans le sérum.

15. Utilisation d'une quantité efficace d'un point de vue thérapeutique d'un agoniste du récepteur de somatostatine de type 5 pour la fabrication d'un médicament pour traiter un sujet affecté par un stimulus qui dépasse l'inhibition dopaminergique normale associé à des taux élevés de prolactine dans le sérum.

16. Utilisation selon la revendication 15, dans laquelle le stimulus qui dépasse l'inhibition dopaminergique normale comprend l'administration d'oestrogène.

17. Utilisation d'une quantité efficace d'un point de vue thérapeutique d'un agoniste du récepteur de somatostatine de type 5 pour la fabrication d'un médicament pour traiter un sujet atteint d'une diminution de l'élimination de la prolactine associée à des taux élevés de prolactine dans le sérum.

18. Utilisation d'une quantité efficace d'un point de vue thérapeutique d'un agoniste du récepteur de somatostatine de type 5 pour la fabrication d'un médicament pour traiter un sujet atteint d'un problème rénal ou de cirrhose associé à des taux élevés de prolactine dans le sérum.

19. Utilisation d'une quantité efficace d'un point de vue thérapeutique d'un agoniste du récepteur de somatostatine de type 5 pour la fabrication d'un médicament pour traiter un sujet atteint de crises associées à des taux élevés de prolactine dans le sérum.

20. Utilisation d'une quantité efficace d'un point de vue thérapeutique d'un agoniste du récepteur de somatostatine de type 5 pour la fabrication d'un médicament pour traiter un sujet atteint d'un trauma de la cage thoracique associé à des taux élevés de prolactine dans le sérum.

21. Utilisation selon la revendication 1, dans laquelle le sujet est un être humain.

22. Utilisation selon la revendication 1, dans laquelle le sujet est atteint d'un prolactinome.

23. Utilisation selon la revendication 22, dans laquelle la quantité d'agoniste du récepteur de somatostatine de type 5 est efficace pour réduire la quantité de prolactine sécrétée par le prolactinome afin de maintenir la prolactine à des taux approximativement normaux dans le sang.

24. Utilisation selon la revendication 22, dans laquelle la quantité d'agoniste à administrer est efficace pour réduire les taux de prolactine dans une fourchette d'environ 2 à environ 30 ng/ml de sang, lorsque mesurés par radiométrie immunologique.

25. Utilisation selon la revendication 22, dans laquelle la quantité d'agoniste du récepteur de somatostatine de type 5 est efficace pour réduire les taux de prolactine dans une fourchette d'environ 5 à environ 20 ng/ml de sang, lorsque mesurés par radiométrie immunologique.

26. Utilisation selon la revendication 22, dans laquelle l'agoniste du récepteur de somatostatine de type 5 présente un Ki pour le récepteur de somatostatine de type 5 qui est inférieur à 1 nM.

27. Utilisation selon la revendication 22, dans laquelle le Ki de l'agoniste sélectif du récepteur de somatostatine de type 5 pour le récepteur de somatostatine de type 5 est au moins 10 fois supérieur au Ki pour le récepteur de somatostatine de type 2.

28. Utilisation d'une quantité efficace d'un point de vue thérapeutique d'un agoniste sélectif du récepteur de somatostatine de type 5 pour la fabrication d'un médicament pour traiter la galactorrhée associée à des taux élevés de prolactine.

29. Utilisation d'une quantité efficace d'un point de vue thérapeutique d'un agoniste sélectif du récepteur de somatostatine de type 5 pour la fabrication d'un médicament pour traiter l'aménorrhée associée à des taux élevés de prolactine.

30. Utilisation d'une quantité efficace d'un point de vue thérapeutique d'un agoniste sélectif du récepteur de somatostatine de type 5 pour la fabrication d'un médicament pour traiter l'infertilité associée à des taux élevés de prolactine.

31. Utilisation d'une quantité efficace d'un point de vue thérapeutique d'un agoniste sélectif du récepteur de somatostatine de type 5 pour la fabrication d'un médicament pour traiter l'impuissance associée à des taux élevés de prolactine.

32. Utilisation d'une quantité efficace d'un point de vue thérapeutique d'un agoniste sélectif du récepteur de somatostatine de type 5 pour la fabrication d'un médicament pour traiter l'hypergonadisme associé à des taux élevés de prolactine.
